# EUROPEAN PATENT SPECIFICATION

(11) **EP 2 570 090 B1**
(45) Date of publication and mention of the grant of the patent: **01.04.2015**
(21) Application number: 11181514.8
(22) Date of filing: 15.09.2011
(51) Int. Cl.: A61B 17/70

(54) **Polyaxial bone anchoring device with enlarged pivot angle**
Mehrachsige Knochenverankerungsvorrichtung mit vergrößertem Schwenkwinkel
Dispositif d'ancrage d'os polyaxial doté d'un angle de pivot élargi

(43) Date of publication of application: 20.03.2013
(73) Proprietor: Biedermann Technologies GmbH & Co. KG, 78166 Donaueschingen (DE)
(72) Inventor: Biedermann, Lutz, 78048 VS-Villingen (DE); Biedermann, Timo, 78647 Trossingen (DE); Dannecker, Berthold, 78112 St. Georgen (DE)
(74) Representative: Prüfer & Partner GbR European Patent Attorneys

(56) References cited:
- EP-A1- 1 923 011
- US-A1- 2005 080 415
- US-A1- 2007 118 123
- US-A1- 2008 015 579
- US-A1- 2010 204 735

## Description

The invention relates to a polyaxial bone anchoring device with an enlarged pivot angle. The polyaxial bone anchoring device includes a bone anchoring element for anchoring in the bone and a receiving part for coupling a stabilization rod to the bone anchoring element. The receiving part comprises a rod receiving portion for receiving the rod and a head receiving portion that is flexible so as to allow introduction and clamping of the head of the bone anchoring element. A locking ring is provided for compressing the head receiving portion of the receiving part to lock the head. A bounding edge of the head receiving portion is configured to permit the anchoring element to pivot at a larger pivot angle at a first location of the bounding edge than at a second location of the bounding edge. The head receiving portion is rotatable with respect to the rod receiving portion so that the orientation of the larger pivot angle can be selected.

A polyaxial bone anchoring device with an enlarged pivot angle is described in US 6,736,820. This bone anchoring device comprises a bone screw and a receiving part with a seat for the head of the bone screw. The screw member can be pivoted to at least one side by an enlarged angle, because the edge of the free end of the receiving part is of asymmetric construction.

Another polyaxial bone anchor is described in US 2005/0080415 A1. This bone anchor has a body member having a U-shaped channel for receiving the rod and a compressible recess for receiving a head of the anchor member such that the anchor member can initially polyaxially angulate with respect to the body member and further has a collar slidably disposed about the body member and capable of compressing the recess around the head. The lower bounding edge of the body member may include a countersunk region to permit increased angulation when the anchor member is oriented toward the countersunk region.

US 2007/0118123 A1 describes a polyaxial bone anchor with increased angulation. The polyaxial bone anchor has a locking element shaped and configured to allow an anchoring member, e.g. a screw or a hook, to polyaxially rotate at large angles about a central axis of the bone anchor before compression locking the anchoring member within an anchor head.

Although the polyaxial bone anchoring devices described above provide for enlarged angulation in a specific orientation, there is still a need for an improved polyaxial bone anchoring device in terms of simplicity of the design and variety of the applications.

US 2010/0204735 A1 describes a coupling assembly with a yoke device that allows an anchor member secured thereto to rotate in a turret-like manner with respect to an elongate member secured by the yoke in addition to allowing the anchor member to pivot away from the central axis of the yoke. The yoke has upper and lower yoke members. The lower yoke member has a slot opening determining the direction of pivotability of the screw shank. The lower yoke member can be rotated so that the slot opening faces the direction in which the screw shank is to be pivoted.

US 2008/0015579 A1 describes bone anchor assemblies having a large diameter for fixing a spinal connection element to bone. The assembly includes a receiver member for receiving the spinal connection element, a bone-engaging shank for engaging bone, a retaining member for retaining the head of the shank within the receiver member and a locking member for locking the retaining member within the receiver member. A retaining member retains the head of the shank within the receiver member. The retaining member may be in the form of a C-shaped ring.

EP 1 923 011 A1 describes a bone anchoring device comprising a receiving part for receiving a rod and an anchoring element and a locking element that may comprise pins inserted into through holes formed in the receiving part and engaging a portion of the anchoring element, such that the anchoring element is pivotable relative to the receiving part around a single axis of rotation.

It is the object of the invention to provide a polyaxial bone anchoring device with an enlarged pivot angle that has a small size by simultaneously providing a safe locking and that can be used as a modular system.

The object is solved by a polyaxial bone anchoring device according to claim 1. Further developments are given in the dependent claims.

The polyaxial bone anchoring device is a bottom loading polyaxial bone anchoring device wherein the anchoring element can be inserted into the receiving part from the bottom of the receiving part. The bone anchoring device can be delivered by the manufacturer as pre-assembled receiving part with locking ring and separate therefrom, one or a plurality of bone anchoring elements. By means of this, various shanks with a different diameter, thread form or other different features can be combined with a receiving part according to the actual clinical requirements in a particular clinical situation. This gives the surgeon a substantial choice of implants.

By the modularity, the costs of stock-holding can be decreased.

Because the enlarged pivot angle can be selected within a range of 360° around the central axis of the receiving part, the bone anchoring device can be used in a variety of applications. The maximum pivot angle of the bone anchoring element relative to the receiving part is equal to or greater than 45° measured from the straight position. The orientation of the enlarged pivot angle can be selected, for example in a plane including the rod axis and the shank axis or at 90° with respect to the rod axis or at any other angle. This renders the bone anchoring device particularly suitable for the application of lateral mass fixation, for example the cervical spine.

The design of the bone anchoring device allows to further reduce the dimension in terms of height as well as in terms of diameter which makes it particularly suitable for applications where small-sized anchoring devices are required, such as in the field of cervical spine surgery or paediatric applications, trauma and minimally open applications for bone surgery.

Further features and advantages will become apparent from the description of embodiments by means of accompanying drawings.

In the drawings:
- Fig. 1:: shows an exploded perspective view of the polyaxial bone anchoring device according to a first embodiment.
- Fig. 2:: shows a perspective view of the polyaxial bone anchoring device of Fig. 1 in an assembled state.
- Fig. 3:: shows a cross-sectional view of the polyaxial bone anchoring device, the section taken perpendicular to the rod axis, in a first pivot position of the bone anchoring element.
- Fig. 4:: shows a cross-sectional view of the polyaxial bone anchoring device, the section taken perpendicular to the rod axis, in a second pivot position of the bone anchoring element.
- Fig. 5:: shows a perspective view of the rod receiving portion of the receiving part of the polyaxial bone anchoring device according to Fig. 1.
- Fig. 6:: shows a perspective view from below of the rod receiving portion of the receiving part.
- Fig. 7:: shows a cross-sectional view of the rod receiving portion of the receiving part, the section taken perpendicular to the rod axis.
- Fig. 8:: shows a side view of the rod receiving portion of the receiving part.
- Fig. 9:: shows a side view rotated by 90° of the rod receiving portion of the receiving part.
- Fig. 10:: shows a top view of the rod receiving portion of the receiving part.
- Fig. 11:: shows a perspective view from below of the head receiving portion of the receiving part of the polyaxial bone anchoring device of Fig. 1.
- Fig. 12:: shows another perspective view from above of the head receiving portion of the receiving part.
- Fig. 13:: shows a cross-sectional view of the head receiving portion of the receiving part.
- Fig. 14:: shows a side view of the head receiving portion of the receiving part.
- Fig. 15:: shows a side view of the receiving part of Fig. 14 rotated by 90°.
- Fig. 16:: shows a view from the top of the head receiving part of the receiving part.
- Fig. 17:: shows an exploded perspective view of the receiving part with steps of assembly (without locking ring).
- Fig. 18:: shows a perspective view of the assembled rod receiving portion and head receiving portion of the receiving part.
- Fig. 19:: shows a perspective view of the locking ring.
- Fig. 20:: shows a cross-sectional view of the locking ring, the section taken along line A-A in Fig. 19.
- Fig. 21:: shows an exploded perspective view of the receiving part without locking ring of the polyaxial bone anchoring device according to a second embodiment.
- Fig. 22:: shows a perspective view of the receiving part without locking ring of Fig. 21 in an assembled state.
- Fig. 23:: shows a cross-sectional view of the assembled receiving part of Fig. 21 without locking ring, the section taken perpendicular to the rod axis.
- Fig. 24:: shows a perspective exploded view of the receiving part without locking ring according to a third embodiment of the polyaxial bone anchoring device.
- Fig. 25:: shows a perspective view of the receiving part of Fig. 24 in an assembled state.
- Fig. 26:: shows a cross-sectional view of the receiving part of Fig. 24, the section taken perpendicular to rod axis.

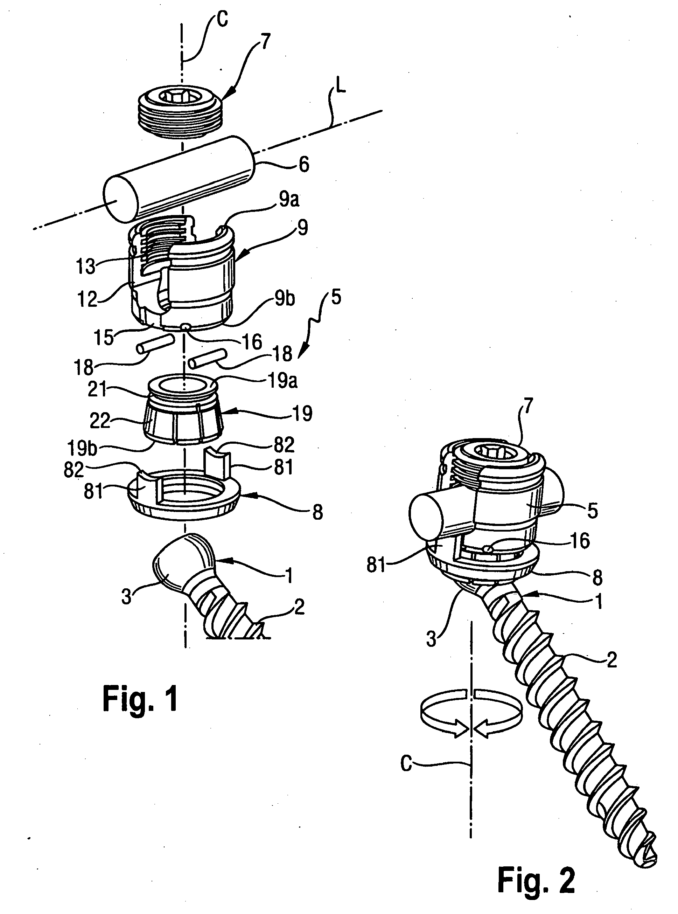

As shown in Figs. 1 to 4, the polyaxial bone anchoring device according to a first embodiment comprises a bone anchoring element 1 in the form of a bone screw having a threaded shank 2 and a spherical segment-shaped head 3. The head 3 has a recess 4 for engagement with a tool. The bone anchoring device further includes a receiving part 5 for receiving a rod 6 to connect it to the bone anchoring element 1. Further, a closure element 7 in the form of an inner screw or set screw is provided for securing the rod 6 in the receiving part 5. In addition, the bone anchoring device comprises a locking ring 8 for locking the head 3 in the receiving part 5.

As can be further seen in Figs. 1 to 16, the receiving part 5 comprises a rod receiving portion 9 and a head receiving portion 19 that are rotatably connected to each other. The rod receiving portion 9 is substantially cylindrical and has a first end 9a and an opposite second end 9b and a central axis of symmetric C going therethrough. The rod receiving portion 9 has a coaxial first bore 10 provided at the second end 9b. The diameter of the first bore 10 is smaller than the diameter of the head 3 of the bone anchoring element. The rod receiving portion 9 also comprises a coaxial second bore 11 extending from the first end 9a to a distance from the second end 9b and a coaxial third bore 11 a being in communication with the first bore 10 and the second bore 11. The diameter of the second bore 11 is larger than than the diameter of the rod 6. A substantially U-shaped recess 12 is provided in the rod receiving portion 9 that extends from the first 9a end to the second end 9b, the diameter of the U-shaped recess being slightly larger than the diameter of the rod to such an extent that the rod 6 can be placed in the recess and is guided therein. By means of the U-shaped recess 12 to free legs 12a, 12b are formed on which an internal thread 13 is provided. The internal thread 13 can be a metric thread, a flat thread, a negative angle thread, a saw-tooth thread or can have any other thread form. Preferably, a thread form such as a flat thread or a negative angle thread is used which prevents splaying of the legs 12a, 12b when the inner screw 7 is screwed-in. The height of the U-shaped recess is such that the rod 6 and the inner screw 7 can be inserted between the legs. Between the bottom of the recess 12 and the legs 12a, 12b, a flat section 14 is provided forming the end of the second bore 11.

At the second end 9b, two cut-outs 15 located diametrically opposite to each other are provided. The cut-outs 15 extend from the second end 9b into the bottom of U-shaped recess 12. The cut-outs 15 are configured to receive a portion of the locking ring 8 described below.

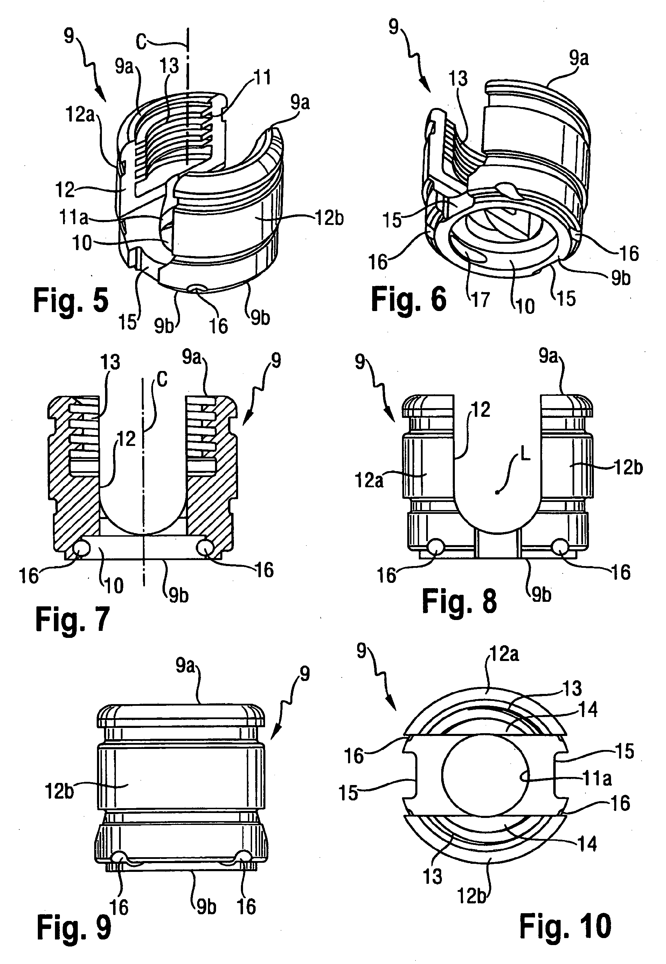

On either side of the cut-outs a pin hole 16 is provided that extends through the rod receiving portion 9 of the receiving part 5 to in parallel to the longitudinal axis L of the channel formed by the recess 12. The pine holes 16 have such a size that they project into the first bore 10 along a circumferential length thereby forming an opening 17, as shown in particular in Fig. 6. As depicted in Figs. 1 to 4 two pins 18 are provided at are configured to be inserted into the pin holes 16, respectively. The length of the pins 18 is such that in the inserted position they do not project out of the outer surface of the rod receiving portion 9 of the receiving part 5. When the pins 18 are inserted a portion 18a of the pin projects through the opening 17 into the first bore 10, thereby reducing the diameter of the first bore 10 at the position of the opening 17. The pins have a circular cross-section. Therefore, the portion 18a that projects through the opening has substantially the shape of a segment of an ellipsoid.

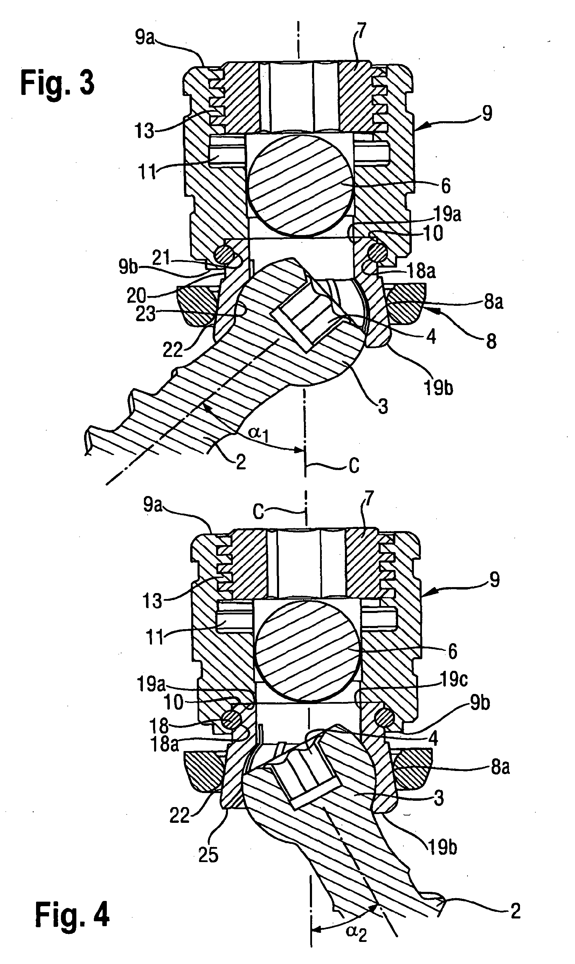

The head receiving portion 19 of the receiving part provides an accommodation space for the head 3 of the bone anchoring element 1. The head receiving portion 19 has a first end 19a facing the second end 9b of the rod receiving portion 9 and a second end 19b and a coaxial through hole 19c. Adjacent to the first end 19a is a substantially cylindrical portion 20 with a circumferential groove 21, The outer diameter of the cylindrical portion 20 is the same as or is slightly smaller than the inner diameter of the first bore 10 of the rod receiving portion 9 of the receiving part 5 so that the cylindrical portion 20 fits into the first bore 10. The diameter of the groove 21 corresponds substantially to the inner diameter of the first bore 10 at the opening 17 when the pins 18 are inserted, as shown in particular in Figures 3 and 4.

The head receiving portion 19 further has a conically-shaped outer surface portion 22 that widens towards the second end 19b. In addition, an internal hollow spherical section 23 forming an accommodation space for the spherical segment-shaped head 3 of the bone anchoring element 1 is formed in the head receiving portion 19. The internal hollow spherical section 23 is configured to encompass the head of the bone anchoring element from the side covering a region including the largest diameter of the head 3.

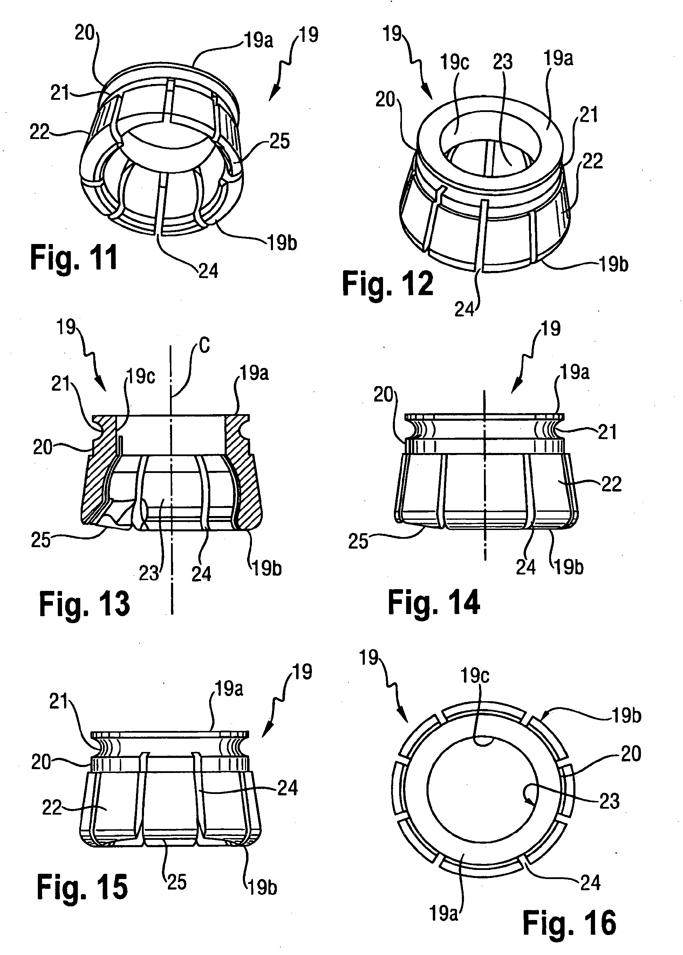

As can be seen in particular in Figs. 11 to 16, a plurality of slits 24 are provided that are open to the second end 19b of the second portion. The slits 24 end at a distance from the second end 19b. By the size an number of the slits a desired elasticity is given to the head receiving portion. The elasticity of the head receiving portion 16 is such that the head 3 of the anchoring element 1 can be inserted by expanding the head receiving portion and can be clamped by compressing the head receiving portion 24.

The edge bounding the second end 19b is asymmetric. In the embodiment shown, this is achieved by a countersunk or recessed area 25 at the internal hollow space 23. By means of this, the anchoring element can pivot at the position of the recessed area 25 to a larger pivot angle α₁ with respect to the straight position when the anchor axis of the anchoring element 1 is coaxial to the central axis of C of the receiving part 5 (Fig. 3) compared to a smaller pivot angle α₂ in the opposite direction (Fig. 4). The recessed area 25, therefore, defines the position of the enlarged pivot angle with respect to the rod receiving portion 9.

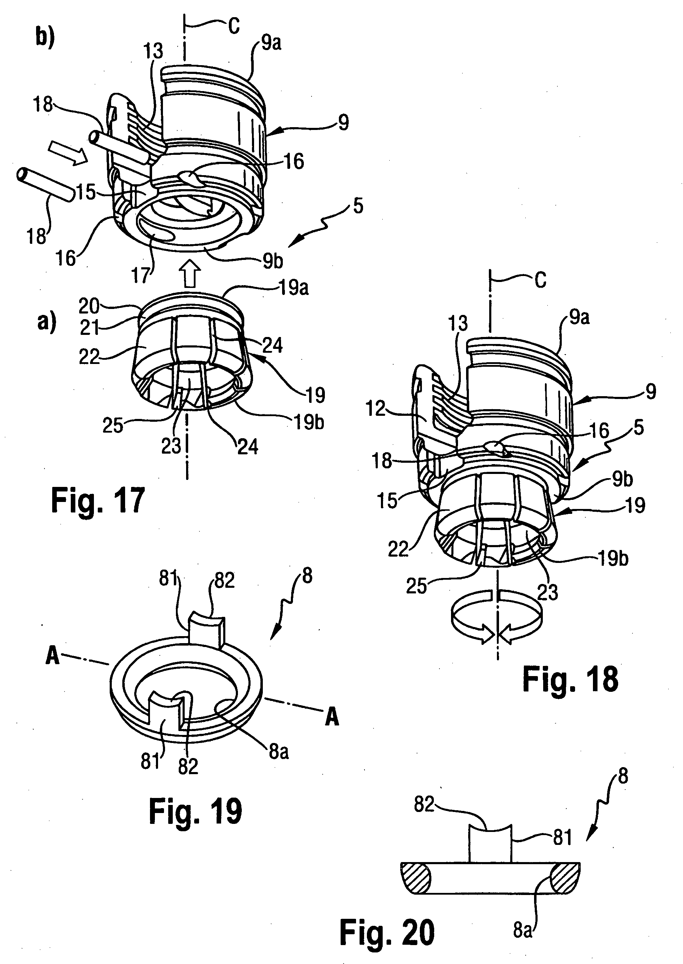

The receiving part 5 is assembled as shown in Fig. 17 and 18. First, in step a) the head receiving portion 19 is introduced into the rod receiving portion from the second end 9b of the first portion, until it abuts with its first end 19a the end of the first bore 10 (Figs. 3 and 4). Then, in step b) the pins 18 are introduced into the pin holes 16 so that they extend partially through the openings 17 into the groove 21. By means of this, the head receiving portion 19 is connected to the rod receiving portion 9 so that it can not fall out. Because the pins 18 extend through the openings 17 like a segment of an ellipsoid, the pins are able to move in the groove 21. This renders the head receiving portion 19 rotatable with respect to the rod receiving portion 9. The orientation of the recessed area 25 with respect to the orientation of the rod axis can be selected at any angle between zero and 360° by rotating the head receiving portion 19 with respect to the rod receiving portion 9 in clockwise or counter clockwise direction.

The locking ring 8 will now be described with reference to Figs. 19 and 20. The locking ring 8 has a substantially cylindrical outer surface with an outer diameter that does not project or only slightly projects outward from the outer surface of the rod receiving portion 9 of the receiving part 5. The height of the locking ring 8 in an axial direction is smaller than that of the head receiving portion 19 of the receiving part 5 so that, as shown in Figs. 3 and 4, there is a distance between the locking ring 8 and the second end 9b of the rod receiving portion 9 when the locking ring 8 is in a position clamping the head 3.

The locking ring 8 has an its inner side a curved internal surface portion 8a. The curvature is directed to the center of the locking ring. The curved surface portion 8a can have a spherical curvature. Other types of curvatures are also possible. The inner diameter of the locking ring is such that the locking ring 8 can slide along the outer conical surface portion 22 of the head receiving portion 19, thereby compressing the head receiving portion 19 increasingly when sliding downwards.

Furthermore, the locking ring 8 comprises on its side facing the second end 9b to projections 81 located diametrically opposite to each other. The projections 81 have such a height that they project above the bottom of the substantially U-shaped recess 12 and extend into the cut-outs 15 when the locking ring 8 is in a position in which the head 3 is not yet clamped. The free ends 82 of the projections can be curved, particularly concavely curved with a curvature that may correspond to that of the rod 6. The locking ring is arranged in such a way around the head receiving portion 19 of the receiving part 5 that the projections 81 are located at the positions of the recess 12. By means of this, the projections 81 that project through the cut-outs 15 into the recess 12 prevent the locking ring 8 from rotating when the rod is not inserted.

The locking ring 8 is mounted from the second end 19b of the second portion. When it is in an upper most position abutting against the second end 9b, the head receiving portion 19 is still freely rotatable with respect to the rod receiving portion 9.

The flexibility of the head receiving portion 19 and the size of the head receiving portion 19 at the open second end 19b allows to mount the locking ring 8 by assembling it from the second end 19b onto the head receiving portion 19. Because the outer diameter of the head receiving portion 19 is smaller than that of the rod receiving portion 9, the locking ring 8 only minimally projects beyond the rod receiving portion in a radial direction.

The locking ring 8 is moveable between a first position limited by the second end 9b of the rod receiving portion 9 of the receiving part that act as a stop and a second position near the second end 19b of the head receiving portion 19. In the second position the head 3 is locked by means of compression of the head portion. The tapered exterior surface 22 of the head receiving portion 19 prevents escaping of the locking ring 8 in direction of the second end 19b.

The inner screw 7 has a thread corresponding to the internal thread 13 provided on the legs. If a thread form that prevents the legs from splaying is used, a single closure element such as the inner screw 7 is sufficient. This reduces the size of the bone anchoring device in radial direction.

The receiving part 5, the locking ring 8, the inner screw 7 and the bone anchoring element 1 are made of bio-compatible material, for example of titanium or stainless steel or of bio-compatible alloys, such as nickel titanium alloys, for example Nitinol, or of a bio-compatible plastic material, such as, for example, polyetheretherketone (PEEK). The parts can be made of the same or of different materials.

The bone anchoring device can be used in several ways. In one way of use, the bone anchoring element 1, the receiving part 5 and the locking ring 8 are pre-assembled. The head receiving portion 19 of the receiving part is rotated to a desired position such that the recessed area 25 that defines the orientation of the enlarged pivot angle, is position at the desired orientation. The bone anchoring element is then inserted into the bone with the receiving part mounted onto the bone anchoring element. The recess 4 of the head can be accessed with a tool through the first, second and third bore. The locking ring is in a first position close to the second end 9b of the rod receiving portion 9 where it does not clamp the head 3. The flexible head receiving portion 19 of the receiving part 5 creates a slight pretension having a small overlap on the inner surface of the hollow portion 23. Therefore, the receiving part 5 is fictionally held on the head 3 in a specific angular position. The receiving part 5 can then be aligned manually to receive the rod 6. Once the correct position of the rod with respect to other bone anchoring devices is achieved, the inner screw 7 is screwed between the legs until it presses onto the rod 6. The rod is pressed into the bottom of the U-shaped recess, thereby engaging the free ends 82 of the projections 81, respectively, and shifting down the locking ring 8. When the locking ring 8 is moved towards the second end 19b of the head receiving portion 19, it compresses the head receiving portion 19 and clamps the head 3. Final tightening of the inner screw locks the rod and the head simultaneously.

In another way of use, only the receiving part 5 and the locking ring 8 are pre-assembled. The bone anchoring element 3 is selected from a variety of bone anchoring elements and introduced into the hollow internal portion 23 while the locking ring is in its position close to the second end 9b of the rod receiving portion. This allows to select the appropriate bone anchoring element out of the variety of the bone anchoring elements that may be differ in diameter, length and other features of the anchoring section. Hence, a modular system is provided including receiving parts and a plurality of bone anchoring elements which can then be than an individually selected. It is also possible to provide plurality of receiving parts with different recessed areas 25 for various enlarged pivot angles. By means of the modularity the field of application of the bone anchoring device is enlarged.

In yet another way of use the inner screw is tightened to lock the head and the rod. Thereafter, the inner screw is loosened to allow further adjustments of the rod. The head remains temporarily clamped due to the frictional force that holds the locking ring 8 in place.

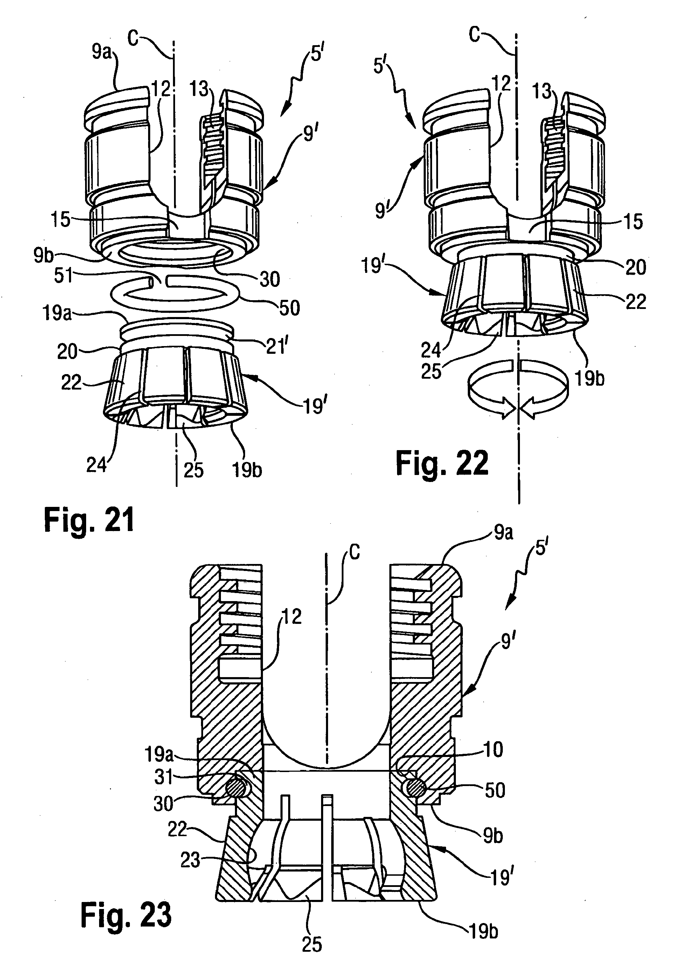

Figs. 21 to 23 show a second embodiment of the polyaxial bone anchoring device that differs from the first embodiment by the receiving part. The receiving part 5' according to the second embodiment comprises a rod receiving portion 9' for receiving the rod 6 and a head receiving portion 19' for receiving the head 3 of the bone anchoring element 1. Portions and sub-portions that are identical to that of the first embodiment are indicated with the same reference numerals and the description thereof will not be repeated. The rod receiving portion 9' does not have the pin holes 16 and the pins 18. The rod receiving portion 9' comprises a circumferential groove 30 at a distance from the second end 9b. The head receiving portion 19' comprises near the first end 19a a circumferential groove 21' for accommodating a split ring 50. The split ring 50 is a ring with a circular cross section that substantially corresponds to the cross section of the groove 30 and that is open i.e. has a gap 51. Therefore, the split ring 50 exhibits flexibility. The size of the split ring 50 is such that if the gap 51 is closed by compressing the split ring 50, the split ring 50 is contained fully within the groove 21'. The grooves 21' and 30 face each other when the head receiving portion 19' is inserted into the rod receiving portion 9'. The size of the grooves 21', 30' is such that when the grooves face each other, there is a space 31 allowing the split ring 50 to expand therein.

The assembly of the receiving part 5' is as follows. First, the split ring 50 is compressed and inserted into the groove 21' of the head receiving portion 19'. Than, the head receiving portion 19' together with the split ring 50 is inserted from the second end 9b into the rod receiving portion 9'. When the first end 19a abuts against the end of the first bore 10, the grooves 21', 30 face each other and the split ring 50 expands. By means of this, the head receiving portion 19' is prevented from falling out. Simultaneously, the head receiving portion 19' is still rotatable with respect to rod receiving portion 9' to allow the recessed area 25 to be rotated to a desired position.

The mounting of the locking ring 8 and the assembly of the whole anchoring device is the same as in the first embodiment.

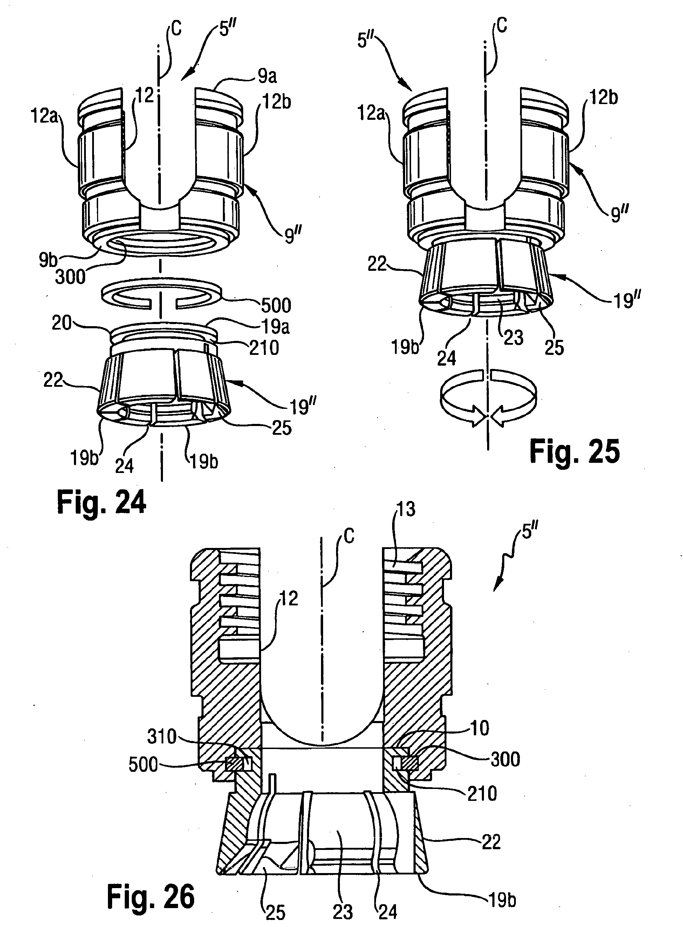

A third embodiment of the polyaxial bone anchoring device is shown in Figs. 24 to 26. The third embodiment differs in the design of the receiving part 5" and the split ring 50' from the second embodiment. Portions and sub-portions that are the same as of the second embodiment are indicated with the same reference numerals in the description thereof will not be repeated.

The third embodiment has a split ring 500 with a rectangular cross section. Also, a groove 300 in the rod receiving portion and a groove 210 in the head receiving portion have rectangular cross section with the long side perpendicular to the central axis. The rectangular cross-section provides a stronger holding force compared to the second embodiment.

Assembly and use of the bone anchoring device according to the third embodiment is similar to the assembly and use of the polyaxial bone anchoring device according to the first and the second embodiment. The split ring 500 is inserted into the groove 210 and compressed.

When the head receiving portion 19" is mounted to the rod receiving portion 9" the split ring 50' expands into the groove 300 because the dimensions of the grooves are such that there is a gap 310. Due to the rectangular cross-section of the split ring 500 and the grooves 210 and 300, the greater friction force during rotation of the head receiving portion allows to move precisely adjust the position of the recessed area 25.

Further modifications of the embodiments described are conceivable. For example, for the bone anchoring element, all kinds of anchoring elements can be used and combined with the receiving part. Theses anchoring elements are e.g. screws of different length, with different diameters, cannulated screws, screws with different thread forms, nails, hooks, etc. The head and the shank may also be separate parts that are connectable to each other.

Modifications of the receiving part are also possible. For example, to generate the enlarged pivot angle it is possible to use a symmetric head receiving portion that has been cut at the bottom in an inclined manner so as to generate an enlarged pivot angle over a larger circumferential area. The recessed area can also be realized by a cut-out in a symmetrical head receiving portion that provides an opening perpendicular to the central axis C.

The rotational support of the head receiving portion in the rod receiving portion can be realized in other ways. The pins in the first embodiment and the corresponding pin holes can be placed at another location in the circumferential direction. One single pin hole and single pin may be sufficient. Instead of the pins or the split ring, for example, it is possible to use a ball bearing. The connection between the rod receiving portion and the head receiving portion is at least partially a positive-fit connection. However, it is also conceivable, that the connection is achieved by a friction-fit connection only.

The configuration of the locking ring and the cooperating outer surface portion of the head receiving portion can be different from the embodiments shown. For example, it is possible that the inner surface of the locking ring is also tapered and cooperates with the tapered outer surface of the second portion. The cooperating surfaces of locking ring and head receiving portion can also be parallel so that the clamping of the head is achieved by an interference fit between the locking ring and the second portion. Any other configuration that generates sufficient locking force for locking the head is conceivable.

Instead of the U-shaped recess for receiving the rod, a recess that is open to the side can be used or the channel for the rod may be closed. Other kinds of locking devices including outer nuts, outer caps, bayonet locking devices or others are possible.

## Claims

1. A polyaxial bone anchoring device, the polyaxial bone anchoring device including
a bone anchoring element (1) having a shank (2) for anchoring in the bone and a head (3);
a receiving part (5, 5', 5 ") for coupling a rod (6) to the bone anchoring element (1); the receiving part including
a rod receiving portion (9, 9', 9") having a first end (9a) and a second end (9b) and a recess (12) with a bottom for receiving the rod (6) therein,
a head receiving portion (19, 19', 19") having a first end (19a) and an open second end (19b) with a bounding edge and a hollow interior portion (23) in communication with the open second end (19b) for introduction of the head (3), the head receiving portion being flexible so as to allow introduction and clamping of the head (3);
a locking ring (8) mounted around the head receiving portion (19);
wherein the head (3) is pivotable in the head receiving portion (19) and can be locked at an angle by compressing the head receiving portion (19) by means of the locking ring (8);
wherein the bounding edge is configured to permit the anchoring element (1) to pivot at a larger pivot angle at a first location of the bounding edge (19c) than at a second location of the bounding edge;
and wherein the head receiving portion (19, 19', 19") is rotatably connected to the rod receiving portion (9, 9', 9"),
**characterized in that** the head receiving portion (19, 19', 19") comprises a plurality of slits (24) that are open to the second end (19b) and wherein the flexibility and the size of the head receiving portion (19, 19', 19") is such that the locking ring (8) is mountable from the open end (19b) of the head receiving portion.

2. The polyaxial bone anchoring device of claim 1, wherein the head receiving portion (19, 19', 19") is connected with its first end (19a) to the second end (9b) of the rod receiving portion (9, 9', 9").

3. The polyaxial bone anchoring device of claim 1 or 2, wherein the head receiving portion (19, 19', 19") and the rod receiving portion (9, 9', 9") are connected through a positive-fit connection.

4. The polyaxial bone anchoring device of one of claims 1 to 3, wherein the head receiving portion (19, 19', 19") has a cylindrical portion (20) at its first end (19a) with a circumferential groove (21) and the rod receiving portion (9, 9', 9") has a bore (10) at its second end (9b) into which the cylindrical portion (20) fits.

5. The polyaxial bone anchoring device of claim 4, wherein the rod receiving portion (9) comprises at least one pin hole (16) and a pin (18) that is positioned so as to extend at least partially into the groove (21).

6. The polyaxial bone anchoring device of claim 4, wherein the bore (10) comprises a circumferential groove (30, 300) in its wall facing the groove (21) of the head receiving portion (19', 19") and wherein a split ring (50, 500) is provided in the groove (21) of the head receiving portion that can extend into the groove (21',210) of the rod receiving portion (9', 9").

7. The polyaxial bone anchoring device of one of claims 1 to 6, wherein the bounding edge comprises a recessed area (25) that provides for an enlarged pivot angle when the bone anchoring element pivots into it.

8. The polyaxial bone anchoring device of one of claims 1 to 7, wherein the head receiving portion (19, 19', 19") is fixed with respect to the rod receiving portion (9, 9', 9") when the locking ring (8) compresses the head receiving portion such that the head is locked.

9. The polyaxial bone anchoring device of one of claims 1 to 8, wherein the receiving part (5, 5', 5") comprises an axis of symmetry (C) that extends from the first end (9a) of the rod receiving portion (9, 9', 9") to the open second end (19b) of the head receiving portion (19, 19', 19") and wherein the locking ring (8) is movable along the axis of symmetry (C).

10. The polyaxial bone anchoring device of one of claims 1 to 9, wherein a movement of the locking ring (8) by exerting pressure onto it through the rod (6) compresses the head receiving portion until the head (3) is locked.

11. The polyaxial bone anchoring device of one of claims 1 to 10, wherein the recess (12) for the rod (6) is a U-shaped recess.

## Patentansprüche

1. Polyaxiale Knochenverankerungsvorrichtung, wobei die polyaxiale Knochenverankerungsvorrichtung umfasst
ein Knochenverankerungselement (1) mit einem Schaft (2) zum Verankern im Knochen und einen Kopf (3);
ein Aufnahmeteil (5, 5', 5") zum Koppeln eines Stabes (6) an das Knochenverankerungselement (1); wobei das Aufnahmeteil umfasst
einen Stabaufnahmeabschnitt (9, 9', 9") mit einem ersten Ende (9a) und einem zweiten Ende (9b) und einer Ausnehmung (12) mit einem Boden zum Aufnehmen des Stabs (6) darin,
einen Kopfaufnahmeabschnitt (19, 19', 19") mit einem ersten Ende (19a) und einem offenen zweiten Ende (19b) mit einem Begrenzungsrand und einem hohlen Innenabschnitt (23), der mit dem offenen zweiten Ende (19b) in Verbindung steht zum Einführen des Kopfes (3), wobei der Kopfaufnahmeabschnitt flexibel ist, so dass er ein Einführen und Klemmen des Kopfes (3) erlaubt;
einen Verriegelungsring (8), der um den Kopfaufnahmeabschnitt (19) befestigt ist;
wobei der Kopf (3) in dem Kopfaufnahmeabschnitt (19) schwenkbar ist und unter einem Winkel durch Zusammendrücken des Kopfaufnahmeabschnitts mittels des Verriegelungsringes (8) verriegelt werden kann;
wobei der Begrenzungsrand ausgebildet ist es dem Verankerungselement (1) zu ermöglichen an einer ersten Stelle des Begrenzungsrandes (19c) um einen größeren Schwenkwinkel zu schwenken als an einer zweiten Stelle des Begrenzungsrandes;
und wobei der Kopfaufnahmeabschnitt (19, 19', 19") drehbar an dem Stabaufnahmeabschnitt (9, 9', 9") befestigt ist,
**dadurch gekennzeichnet, dass** der Kopfaufnahmeabschnitt (19, 19', 19") eine Mehrzahl von Schlitzen (24) umfasst, die zum zweiten Ende (19b) hin offen sind, und wobei die Flexibilität und die Größe des Kopfaufnahmeabschnitts (19, 19', 19") derart ist, dass der Verriegelungsring (8) von dem offenen Ende (19b) des Kopfaufnahmeabschnitts her befestigbar ist.

2. Polyaxiale Knochenverankerungsvorrichtung nach Anspruch 1, wobei der Kopfaufnahmeabschnitt (19, 19', 19") mit seinem ersten Ende (19a) mit dem zweiten Ende des Stabaufnahmeabschnitts (9, 9', 9") verbunden ist.

3. Polyaxiale Knochenverankerungsvorrichtung nach Anspruch 1 oder 2, wobei der Kopfaufnahmeabschnitt (19, 19', 19") und der Stabaufnahmeabschnitt (9, 9', 9") durch eine formschlüssige Verbindung verbunden sind.

4. Polyaxiale Knochenverankerungsvorrichtung nach einem der Ansprüche 1 bis 3, wobei der Kopfaufnahmeabschnitt (19, 19', 19") einen zylindrischen Abschnitt (20) mit einer Umfangsnut (21) an seinem ersten Ende (19a) aufweist und der Stabaufnahmeabschnitt (9, 9', 9") eine Bohrung (10) an seinem zweiten Ende (9b) aufweist in welche der zylindrische Abschnitt (20) hinein passt.

5. Polyaxiale Knochenverankerungsvorrichtung nach Anspruch 4, wobei der Stabaufnahmeabschnitt (9) wenigstens ein Stiftloch (16) umfasst und einen Stift (18) der so positioniert ist, dass er sich wenigstens teilweise in die Nut (21) hinein erstreckt.

6. Polyaxiale Knochenverankerungsvorrichtung nach Anspruch 4, wobei die Bohrung (10) eine Umfangsnut (30, 300) in ihrer Wandung, welche der Nut (21) des Kopfaufnahmeabschnitts (19', 19") zugewandten ist, umfasst und wobei ein geschlitzter Ring (50, 500)in der Nut (21) des Kopfaufnahmeabschnitts vorgesehen ist welcher in die Nut (21', 210) des Stabaufnahmeabschnitts (9', 9") hineinragen kann.

7. Polyaxiale Knochenverankerungsvorrichtung nach einem der Ansprüche 1 bis 6, wobei der Begrenzungsrand einen ausgesparten Bereich (25) umfasst der einen vergrößerten Schwenkwinkel gewährleistet wenn das Knochenverankerungselement in ihn hinein schwenkt.

8. Polyaxiale Knochenverankerungsvorrichtung nach einem der Ansprüche 1 bis 7, wobei der Kopfaufnahmeabschnitt (19, 19', 19") bezüglich des Stabaufnahmeabschnitts (9, 9', 9") fixiert ist wenn der Verriegelungsring (8) den Kopfaufnahmeabschnitt zusammendrückt so dass der Kopf verriegelt ist.

9. Polyaxiale Knochenverankerungsvorrichtung nach einem der Ansprüche 1 bis 8, wobei das Aufnahmeteil (5, 5', 5") eine Symmetrieachse (C) umfasst, die sich von dem ersten Ende (9a) des Stabaufnahmeabschnitts (9, 9', 9") zu dem offenen zweiten Ende (19b) des Kopfaufnahmeabschnitts (19, 19', 19") erstreckt, und wobei der Verriegelungsring (8) an der Symmetrieachse (C) entlang bewegbar ist.

10. Polyaxiale Knochenverankerungsvorrichtung nach einem der Ansprüche 1 bis 9, wobei eine Bewegung des Verriegelungsringes (8) durch Ausüben von Druck durch den Stab (6) auf ihn den Kopfaufnahmeabschnitt zusammendrückt bis der Kopf (3) verriegelt ist.

11. Polyaxiale Knochenverankerungsvorrichtung nach einem der Ansprüche 1 bis 10, wobei die Ausnehmung (12) für den Stab (6) eine U-förmige Ausnehmung ist.

## Revendications

1. Dispositif d'ancrage osseux polyaxial, le dispositif d'ancrage osseux polyaxial comportant
un élément d'ancrage osseux (1) ayant une bande de jambe (2) à ancrer dans l'os et une tête (3) ;
une partie de réception (5, 5', 5") pour coupler une tige (6) à l'élément d'ancrage osseux (1) ; la partie de réception comportant
une partie de réception de tige (9, 9', 9") ayant une première extrémité (9a) et une deuxième extrémité (9b) et un évidement (12) avec un fond pour y recevoir la tige (6),
une partie de réception de tête (19, 19', 19") ayant une première extrémité (19a) et une deuxième extrémité ouverte (19b) avec un bord de délimitation et une partie intérieure creuse (23) en communication avec la deuxième extrémité ouverte (19b) pour l'introduction de la tête (3), la partie de réception de tête étant flexible de manière à permettre l'introduction et le serrage de la tête (3) ;
une bague de verrouillage (8) montée autour de la partie de réception de tête (19) ;
dans lequel la tête (3) peut pivoter dans la partie de réception de tête (19) et peut être verrouillée à un angle par compression de la partie de réception de tête (19) au moyen de la bague de verrouillage (8) ;
dans lequel le bord de délimitation est configuré pour permettre à l'élément d'ancrage (1) de pivoter à un angle de pivotement plus grand au niveau d'un premier emplacement du bord de délimitation (19c) qu'au niveau d'un deuxième emplacement du bord de délimitation ;
et dans lequel la partie de réception de tête (19, 19', 19") est reliée en rotation à la partie de réception de tige (9, 9', 9"),
**caractérisé en ce que** la partie de réception de tête (19, 19', 19") comprend une pluralité de fentes (24) qui sont ouvertes sur la deuxième extrémité (19b) et dans lequel la flexibilité et la taille de la partie de réception de tête (19, 19', 19") sont telles que la bague de verrouillage (8) peut être montée à partir de l'extrémité ouverte (19b) de la partie de réception de tête.

2. Dispositif d'ancrage osseux polyaxial de la revendication 1, dans lequel la partie de réception de tête (19, 19', 19") est reliée avec sa première extrémité (19a) à la deuxième extrémité (9b) de la partie de réception de tige (9, 9', 9").

3. Dispositif d'ancrage osseux polyaxial de la revendication 1 ou 2, dans lequel la partie de réception de tête (19, 19', 19") et la partie de réception de tige (9, 9', 9") sont reliées par l'intermédiaire d'une liaison par complémentarité de forme.

4. Dispositif d'ancrage osseux polyaxial de l'une des revendications 1 à 3, dans lequel la partie de réception de tête (19, 19', 19") a une partie cylindrique (20) au niveau de sa première extrémité (19a) avec une rainure circonférentielle (21) et la partie de réception de tige (9, 9', 9") a un alésage (10) au niveau de sa deuxième extrémité (9b) dans lequel la partie cylindrique (20) s'ajuste.

5. Dispositif d'ancrage osseux polyaxial de la revendication 4, dans lequel la partie de réception de tige (9) comprend au moins un trou de broche (16) et une broche (18) qui est positionnée de manière à s'étendre au moins partiellement dans la rainure (21).

6. Dispositif d'ancrage osseux polyaxial de la revendication 4, dans lequel l'alésage (10) comprend une rainure circonférentielle (30, 300) dans sa paroi faisant face à la rainure (21) de la partie de réception de tête (19', 19") et dans lequel une bague fendue (50, 500) est prévue dans la rainure (21) de la partie de réception de tête qui peut s'étendre dans la rainure (21', 210) de la partie de réception de tige (9', 9").

7. Dispositif d'ancrage osseux polyaxial de l'une des revendications 1 à 6, dans lequel le bord de délimitation comprend une zone en retrait (25) qui fournit un angle de pivotement élargi lorsque l'élément d'ancrage osseux pivote dans celle-ci.

8. Dispositif d'ancrage osseux polyaxial de l'une des revendications 1 à 7, dans lequel la partie de réception de tête (19, 19', 19") est fixe par rapport à la partie de réception de tige (9, 9', 9") lorsque la bague de verrouillage (8) comprime la partie de réception de tête de sorte que la tête soit verrouillée.

9. Dispositif d'ancrage osseux polyaxial de l'une des revendications 1 à 8, dans lequel la partie de réception (5, 5', 5") comprend un axe de symétrie (C) qui s'étend de la première extrémité (9a) de la partie de réception de tige (9, 9', 9") à la deuxième extrémité ouverte (19b) de la partie de réception de tête (19, 19', 19") et dans lequel la bague de verrouillage (8) est mobile le long de l'axe de symétrie (C).

10. Dispositif d'ancrage osseux polyaxial de l'une des revendications 1 à 9, dans lequel un mouvement de la bague de verrouillage (8) en exerçant une pression sur celle-ci à travers la tige (6) comprime la partie de réception de tête jusqu'à ce que la tête (3) soit verrouillée.

11. Dispositif d'ancrage osseux polyaxial de l'une des revendications 1 à 10, dans lequel l'évidement (12) pour la tige (6) est un évidement en forme de U.
